# FASCICULE DE BREVET EUROPEEN

(11) **EP 3 129 154 B1**
(45) Date de publication et mention de la délivrance du brevet: **15.12.2021**
(21) Numéro de dépôt: 15713683.9
(22) Date de dépôt: 25.03.2015
(51) Int. Cl.: B05B 11/00, B05B 11/04, B65D 1/32, B65D 81/24, B65D 47/24

(54) **MODULE DE DISTRIBUTION D'UN PRODUIT DESTINÉ À ÊTRE MONTÉ SUR UN CONDUIT D'AMENÉE SOUS PRESSION DUDIT PRODUIT**
MODUL ZUR ABGABE EINES PRODUKTS ZUR MONTAGE AUF EINEM DRUCKZUFUHRKANAL FÜR DIESES PRODUKT
MODULE FOR DISPENSING A PRODUCT INTENDED TO BE MOUNTED ON A PRESSURIZED SUPPLY DUCT FOR SAID PRODUCT

(30) Priorité: 08.04.2014 FR 1453116
(43) Date de publication de la demande: 15.02.2017
(73) Titulaire: Albéa le Tréport, 76470 Le Tréport (FR)
(72) Inventeur: CARRARO, Daniel, F-92600 Asnières-sur-Seine (FR); DEFERT, Sylvain, F-95310 Saint-Ouen l'Aumône (FR)
(74) Mandataire: Gevers & Orès
(86) Numéro de dépôt international: PCT/EP2015/056480
(87) Numéro de publication internationale: WO 2015/155015

(56) Documents cités:
- EP-A1- 2 881 334
- EP-A1- 2 923 964
- WO-A1-2012/013894
- WO-A1-2012/084557
- GB-A- 2 470 615
- US-A1- 2007 194 149

## Description

L'invention concerne un module de distribution d'un produit ainsi qu'un récipient comprenant un réservoir dans lequel un produit est destiné à être conditionné, ledit récipient présentant un conduit d'amenée sous pression dudit produit et, monté sur ledit conduit, un tel module de distribution.

Dans une application particulière, le produit est de type lotion, gel ou crème, par exemple pour une utilisation en cosmétique ou pour des traitements pharmaceutiques.

Le module de distribution peut comprendre un chapeau qui est monté sur une base en formant entre eux un chemin de restitution du produit depuis un passage d'alimentation destiné à être en communication avec le conduit d'amenée vers un passage de sortie dudit produit en vue de sa distribution, par exemple sous la forme d'une noisette ou d'un flot continu.

Selon une réalisation, le module est alimenté en produit sous pression par déformation manuelle réversible du réservoir, par exemple sous la forme d'un tube souple. Selon une autre réalisation, le module est de type bouton poussoir pour actionner le déplacement du conduit d'amenée d'une pompe sur une course de distribution / aspiration du produit sous pression.

A travers le monde diverses directives visent à réglementer, maîtriser et limiter la présence de substances potentiellement dangereuses pour la santé humaine dans les produits notamment cosmétiques. L'une d'elles est la directive européenne REACh (Registration Evaluation and Authorisation of Chemicals). Aussi, une tendance environnementale pousse les cosméticiens à limiter, voire supprimer de leurs formules les conservateurs qui sont souvent cause d'allergies ou d'intolérances.

Les produits cosmétiques deviennent donc de plus en plus fragiles. En particulier, ils supportent difficilement le stress mécanique ou thermique (causant par exemple un déphasage), le contact de l'air (causant par exemple un dessèchement, une oxydation), et sont facilement contaminables par les bactéries et les champignons.

Pour lutter contre la contamination, les formulateurs tentent de renforcer l'activité conservatrice intrinsèque de leurs produits en ajoutant des ingrédients ayant une activité conservatrice comme certaines huiles essentielles, des essences d'orange, de la vitamine C,... qui ne sont pas déclarés en tant que conservateurs. Aussi, ils limitent l'activité libre de l'eau qu'ils tentent de maintenir basse (AW<0,6) afin que les bactéries ne se développent pas ou peu.

D'un autre côté, tant au niveau du réservoir dans lequel le produit est conditionné qu'au niveau du module de distribution, des récipients protecteurs apparaissent sur le marché. En particulier, les récipients doivent empêcher la contamination microbiologique du produit, non seulement lors du stockage mais surtout entre deux utilisations, et notamment par rétro-contamination depuis le passage de sortie vers l'intérieur du réservoir par l'intermédiaire du chemin de restitution.

On connaît des récipients, divulgués dans les documents WO2012/084557, WO2012/013894, GB2470615, US2007/194149, dont les modules de distribution présentent des volumes où du produit stagne au voisinage de la sortie de distribution, et qui risque d'être contaminé entre deux utilisations.

Pourremédier à cela, on a proposé des modules de distribution complexes et coûteux, dont le haut niveau de protection antimicrobien n'est pas toujours indispensable, notamment lorsque le produit présente à lui seul une activité conservatrice intrinsèque. Parmi ces modules complexes, on connaît des documents EP2881334 (Art. 54(3) CBE) et EP2923964 (Art. 54(3) CBE) ceux fonctionnant avec une membrane flexible qui permet de fermer hermétiquement le chemin de distribution, L'invention vise à perfectionner l'art antérieur en proposant notamment un module de distribution de conception simple dans laquelle la restitution du produit est assurée en évitant sa contamination microbienne entre deux utilisations, notamment par rétro-contamination depuis le passage de sortie vers l'intérieur du réservoir.

A cet effet, selon un premier aspect, l'invention propose un module de distribution selon la revendication 1.

Selon un deuxième aspect, l'invention propose un récipient comprenant un réservoir dans lequel un produit est destiné à être conditionné, ledit récipient présentant un conduit d'amenée sous pression dudit produit et, monté sur ledit conduit, un tel module de distribution.

D'autres objets et avantages de l'invention apparaîtront dans la description qui suit, faite en référence aux figures annexées, dans lesquelles :
- la figure 1 est une vue de dessus d'un récipient selon un mode de réalisation de l'invention ;
- la figure 1a est une vue partielle en coupe selon la ligne AA du récipient de la figure 1 ;
- la figure 1b est une vue en coupe selon la ligne BB du module de distribution du récipient de la figure 1 ;
- les figures 2 représentent le chapeau du module de distribution du récipient de la figure 1, respectivement en perspective de côté (figure 2a) et de dessous (figure 2b) ;
- les figures 3 représentent la base du module de distribution du récipient de la figure 1, respectivement en perspective de côté (figure 3a) et de dessus (figure 3b) ;
- la figure 4 représente une base d'un module de distribution selon une variante de réalisation de l'invention ;
- la figure 5 représente une base d'un module de distribution selon une variante de réalisation de l'invention, la figure 5a étant une vue partielle en coupe longitudinale d'un récipient équipé d'un module de distribution comprenant cette base ;
- les figures 6 sont des vues en perspective coupée d'un récipient selon un mode de réalisation ne faisant pas partie de l'invention, dans lesquelles le module de distribution est respectivement en état fermé (figure 6a) et en état ouvert (figure 6b) ;
- la figure 7 représente partiellement en coupe longitudinale un récipient selon un mode de réalisation de l'invention ;
- la figure 8 représente partiellement en coupe longitudinale un récipient selon un mode de réalisation de l'invention.

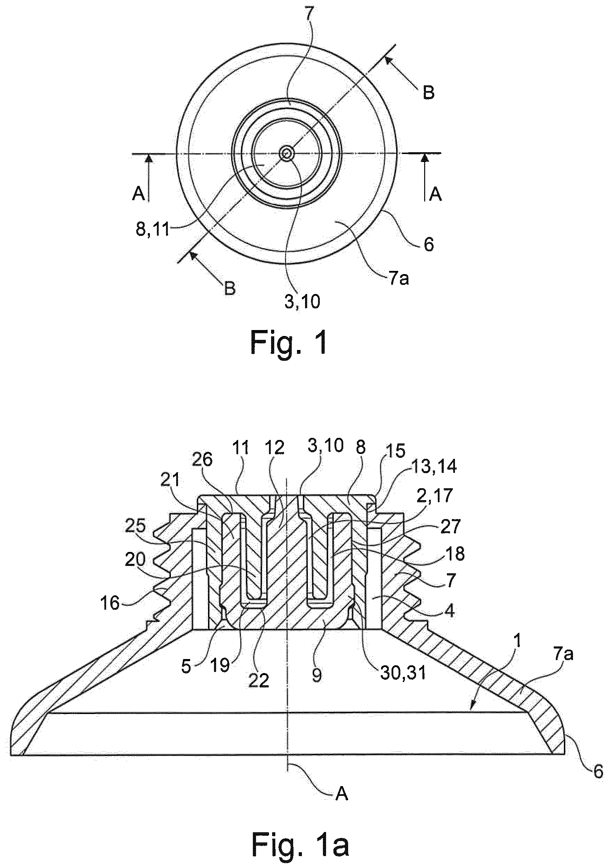

Dans la description, les termes de positionnement dans l'espace sont pris en référence à la position droite du module de distribution et du réservoir telle que représentée sur les figures 1a, 5a et 6 à 8.

En relation avec les figures, on décrit un récipient comprenant un réservoir 1 dans lequel un produit est destiné à être conditionné. Dans un exemple d'application, le produit est une lotion, un gel ou une crème, pour un usage cosmétique ou pour des traitements pharmaceutiques.

Le récipient est équipé d'un module de distribution du produit conditionné dans le réservoir 1, ledit module présentant un chemin de restitution 2 dudit produit depuis ledit réservoir jusqu'à un passage de sortie 3 en vue de sa distribution, par exemple sous la forme d'une noisette ou d'un flot continu. Pour ce faire, le récipient présente un conduit 4 d'amenée sous pression du produit sur lequel le module de distribution est monté en mettant un passage d'alimentation 5 du chemin de restitution 2 en communication avec le réservoir 1.

Dans les modes de réalisation représentés, le récipient présente un corps souple 6 qui est surmonté par une tête 7 formant un conduit 4 d'amenée dans lequel le module de distribution est monté, ledit module étant alimenté en produit sous pression par déformation manuelle réversible du corps 6 du réservoir 1. En particulier, le module de distribution vient occulter la tête 7 en ne permettant la distribution du produit que par l'intermédiaire du chemin de restitution 2, la dimension externe dudit module pouvant être sensiblement analogue à la dimension interne de ladite tête.

Dans les modes de réalisation représentés, le récipient est sous la forme d'un tube composé d'une jupe souple 6 surmontée par une tête 7, ladite tête présentant un goulot dans lequel le conduit d'amenée 4 est formé et une épaule 7a de liaison dudit goulot à la jupe souple 6.

La jupe souple 6 est en général formée d'un film multicouche que l'on enroule sur lui-même, ladite jupe pouvant également être obtenue par extrusion par laquelle un cylindre creux sort de l'extrudeuse et est coupé aux dimensions voulues pour constituer la jupe 6.

La tête 7 de tube peut être moulée par injection directement sur la jupe 6, le moule comprend alors la forme de la tête 7 à mouler et l'extrémité supérieure de la jupe 6 sur laquelle le matériau de la tête 7 va se surmouler, ou moulée par injection de manière séparée. Dans ce dernier cas, la tête 7 de tube est fixée à la jupe 6 par soudage thermique.

Selon une autre réalisation, la tête 7 du récipient est équipée d'un organe de prélèvement sous pression du produit contenu dans le réservoir 1, notamment une pompe intégrant les moyens nécessaires pour la mise sous pression du produit à distribuer par l'intermédiaire d'un conduit d'amenée. En particulier, le conduit d'amenée peut être déplaçable réversiblement sur une course de distribution / aspiration du produit, ledit déplacement étant actionné par un module de distribution formant bouton poussoir.

En relation avec les figures, le module de distribution comprend un chapeau 8 qui est monté sur une base 9 en formant entre eux le chemin de restitution 2 du produit depuis un passage d'alimentation 5 destiné à être en communication avec le conduit d'amenée 4 vers un passage de sortie 3 dudit produit en vue de sa distribution, ledit chemin présentant lesdits passages d'alimentation et de sortie.

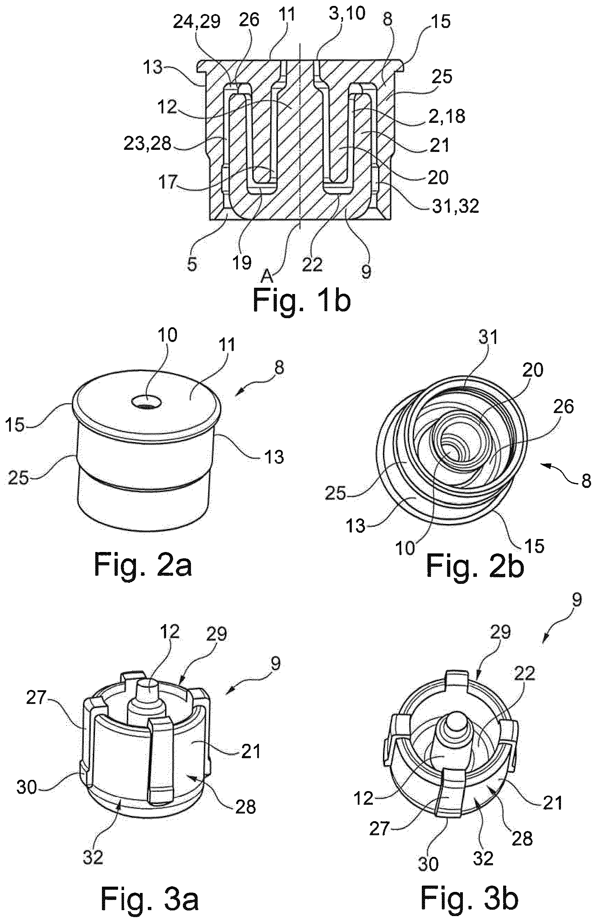

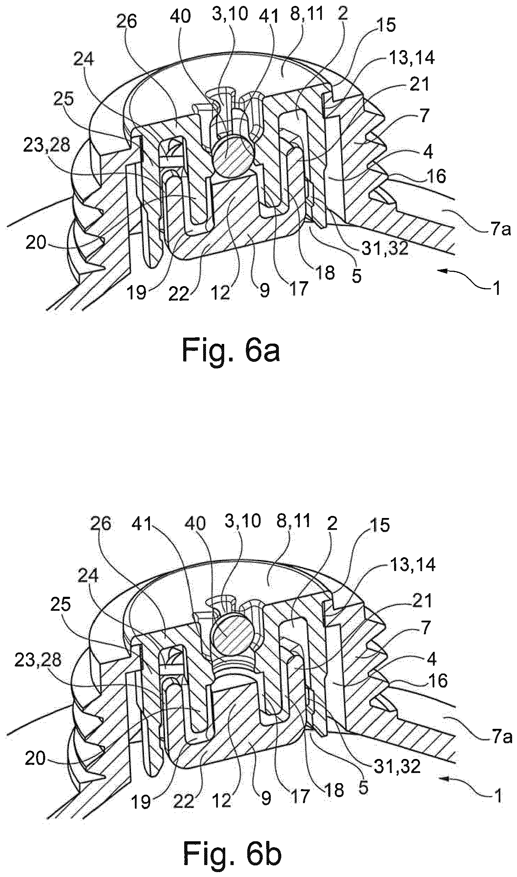

En particulier, le chapeau 8 présente un orifice 10 débouchant dans une paroi supérieure 11, la base 9 présentant un pointeau 12. Selon les modes de réalisations des figures 1 à 5 et 7 à 8, l'extrémité libre du pointeau 12 est disposée dans l'orifice 10 pour former à leur interface le passage de sortie 3. Sur les figures 6, le passage de sortie 3 est équipé d'un clapet sous la forme d'une bille 40 montée dans un siège 41 entre un état stable de fermeture dudit passage (figure 6a) et un état soulevé d'ouverture (figure 6b) par pression du produit afin de permettre sa distribution au travers de l'orifice 10.

Par ailleurs, la base 9 et/ou le chapeau 8 peuvent comprendre des moyens de montage sur le conduit 4 d'amenée sous pression du produit. Dans les modes de réalisation des figures 1 à 6, le chapeau 8 présente une portée extérieure 13 qui est emmanchée dans une couronne 14 formée dans le goulot de la tête 7, ladite portée étant surmontée par un rebord radial 15 venant en appui axial sur ladite couronne pour définir la fin d'emmanchement. Par ailleurs, la tête 7 est pourvue d'un filetage extérieur 16 permettant le montage d'un bouchon de recouvrement du module de distribution entre deux utilisations.

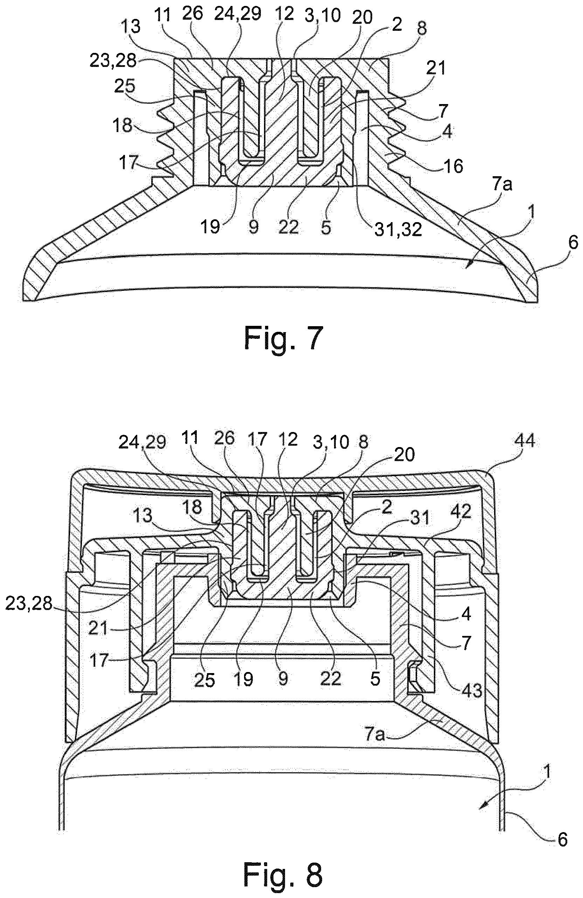

En relation avec la figure 7, la portée extérieure 13 est formée dans le goulot de la tête 7 afin de réaliser d'une seule pièce le chapeau 8 et ladite tête. La figure 8 représente l'intégration du chapeau 8 dans une capsule service, la portée extérieure 13 reliant d'une seule pièce ledit chapeau au manchon 42 de ladite capsule. En particulier, le manchon 42 présente une jupe 43 d'encliquetage autour de la tête 7 et un couvercle 44 monté sur ledit manchon entre deux utilisations pour recouvrir le module de distribution.

Le chemin de restitution 2 présente au moins un conduit aval 17 qui débouche dans le passage de sortie 3 et au moins un conduit amont 18 qui est en communication avec le passage d'alimentation 5. En particulier, les conduits amont 18 et aval 17 sont disposés concentriquement par rapport à un axe A de sortie du produit, le conduit amont 18 étant disposé autour d'au moins une partie du conduit aval 17.

Les conduits amont 18 et aval 17 présentent une jonction 19 en U qui est agencée pour mettre lesdits conduits en communication en occasionnant une perte de charge importante dans le chemin de restitution 2. En particulier, la perte de charge est agencée pour limiter le phénomène de ré-aspiration du produit depuis le passage de sortie 3 vers le réservoir 1 par mémoire de forme du corps 6 après sa déformation pour mettre sous pression le produit. Ainsi, la jonction 19 en U contribue à la protection antimicrobienne entre deux utilisations en évitant que du produit potentiellement contaminé lors de la distribution ne soit introduit dans le chemin de restitution 2 et donc distribué ultérieurement.

Pour renforcer la protection vis-à-vis du risque de contamination du produit par les bactéries et les champignons, au moins l'un des conduits amont 18 et aval 17 est délimité par au moins une paroi qui est apte à assurer une action microbiocide sur le produit contenu dans ledit conduit. Ainsi, même en cas de légère ré-aspiration de produit dans le chemin de restitution 2, celui-ci est décontaminé lors de son immobilisation entre deux utilisations afin de ne risquer ni la distribution ultérieure d'une dose de produit contaminé ni la rétro-contamination du produit contenu dans le réservoir 1.

De façon avantageuse, la paroi supérieure 11 du chapeau 8 peut être apte à assurer une action microbiocide sur le produit disposé sur elle entre deux distributions, notamment sous la forme de souillures par étalement dudit produit sur ladite paroi lors de sa récupération par le doigt de l'utilisatrice.

En particulier, on peut prévoir que le chapeau 8 et/ou la base 9 soient réalisés à base d'un matériau présentant des propriétés microbiocides afin de délimiter au moins un conduit 17, 18 avec au moins une paroi apte à assurer une action microbiocide, notamment une paroi disposée au voisinage immédiat du passage de sortie 3 au niveau duquel le risque d'introduction de contaminants est le plus important. En outre, en relation avec les figures 6, la bille 40 peut être réalisée à base d'un matériau apte à assurer une action microbiocide.

Les propriétés microbiocides du matériau peuvent être obtenues par diffusion dans le produit d'un agent antimicrobien, par exemple sur base organique tel que le Trichlosan (dénomination commerciale de la Sté Melcoplast) ou sur base argent, ou encore minérale. En particulier, le matériau peut comprendre au moins une polyoléfine, par exemple du polyéthylène, du polypropylène et/ou du polystyrène, qui est chargée avec au moins un agent antimicrobien.

Les propriétés microbiocides du matériau peuvent également être obtenues par contact du produit avec un agent microbiostatique, par exemple en utilisant un matériau métallique tel qu'un alliage de Cuivre ou de Zinc ou un matériau comprenant au moins une polyoléfine chargée de telles particules métalliques ou ayant subi un traitement de surface par fluoration, zingage ou cuivrage.

Les propriétés microbiocides du matériau peuvent également être obtenues par irradiation du produit avec un rayonnement de longueur d'onde adaptée, notamment au moyen d'un matériau qui présente des propriétés de photoluminescence après exposition à la lumière extérieure. En particulier, le matériau peut être à base d'au moins une polyoléfine chargée avec au moins un additif apte à émettre un rayonnement photoluminescent qui présente une longueur d'onde comprise entre 250 et 260 nanomètres, et notamment de 254 nanomètres, ce qui correspond à l'ordre de grandeur des rayonnements ultraviolets stérilisants.

Dans les modes de réalisation représentés, la paroi supérieure 11 est équipée d'une jupe intérieure 20 qui s'étend sous l'orifice 10 en étant disposée autour du pointeau 12 pour former le conduit aval 17 à l'interface entre la paroi interne de ladite jupe intérieure et la paroi périphérique dudit pointeau, le conduit amont 18 étant formé à l'interface entre la paroi externe de ladite jupe et la paroi interne d'une cupule 21 de la base 9.

De façon avantageuse, les conduits amont 18 et aval 17 sont chacun délimités entre deux parois dont l'écartement est fonction de la viscosité du produit et de sa sensibilité à la contamination microbienne, par exemple en étant compris entre 0,2 mm et 1 mm, notamment en étant de l'ordre de 0,3 mm.

Ainsi, en réduisant sa quantité, on peut assurer une décontamination fiable et rapide du produit contenu dans le chemin de restitution 2 entre deux distributions. En particulier, l'action microbiocide sur le produit contenu dans le chemin de restitution 2 est agencée pour être plus rapide que la prolifération microbienne vers le réservoir 1, stoppant ainsi sa progression.

La jonction 19 en U est formée entre l'extrémité libre de la jupe intérieure 20 et une portée 22 de raccordement entre le pointeau 12 et la cupule 21, le conduit aval 17 présentant une direction ascendante de circulation du produit depuis la jonction 19 en U vers le passage de sortie 3, le conduit amont 18 présentant une direction descendante de circulation dudit produit. Le chemin de restitution 2 présente ainsi un virage à 180° afin d'augmenter sa longueur sans impacter de façon importante l'encombrement du module de distribution.

En outre, le chemin de restitution 2 présente un deuxième conduit amont 23 par l'intermédiaire duquel le premier conduit amont 18 est en communication avec le passage d'alimentation 5. Pour ce faire, le passage d'alimentation 5 débouche dans le deuxième conduit amont 23 et les deux conduits amont 18, 23 sont en communication par une jonction 24 en U dont la direction est opposée à celle de la jonction 19 en U mettant en communication les conduits amont 18 et aval 17. Ainsi, on augmente encore la longueur du chemin de restitution 2 et donc la perte de charge dans ledit chemin afin de limiter encore plus la possibilité de ré-aspiration de produit contaminé.

Dans les modes de réalisation représentés, la paroi supérieure 11 est équipée d'une jupe extérieure 25 s'étendant autour de la jupe intérieure 20, la portée 13 étant formée sur la paroi externe de ladite jupe extérieure. Le deuxième conduit amont 23 est formé à l'interface entre la paroi externe de la cupule 21 et la paroi interne de la jupe extérieure 25, la deuxième jonction 24 en U étant formée entre l'extrémité libre de la cupule 21 et une portée 26 de raccordement entre lesdites jupes.

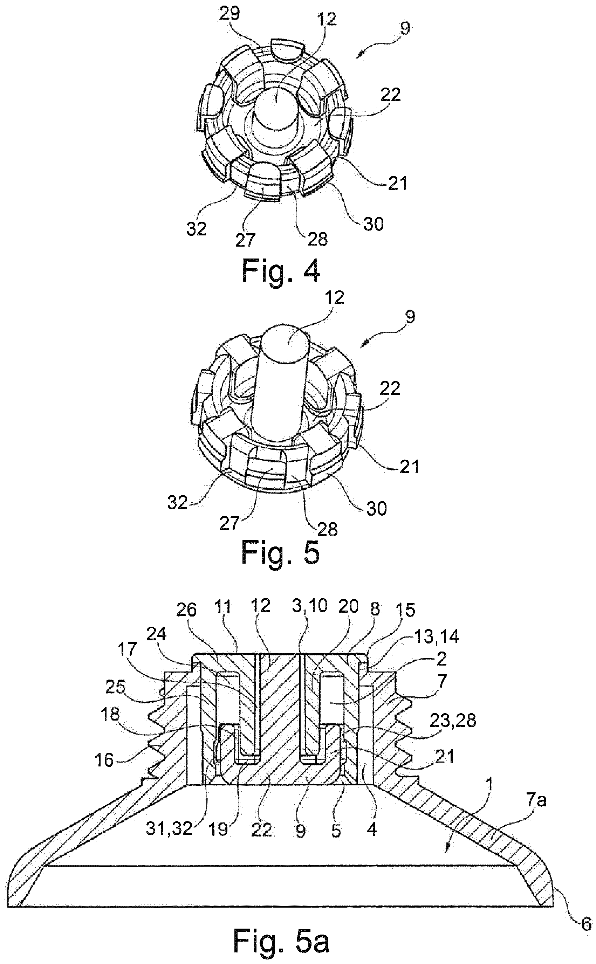

En relation avec les figures 5 et 6, la dimension axiale de la cupule 21 est inférieure à celle du pointeau 12 et des jupes 20, 25 afin d'augmenter la contenance en produit au niveau de la jonction 24 en U. Ainsi, on limite le phénomène de ré-aspiration du produit en formant un réservoir tampon dans le chemin de restitution 2 entre le passage de sortie 3 et le réservoir 1, le produit contenu dans ledit réservoir pouvant alors être décontaminé entre deux utilisations.

La paroi externe de la cupule 21 est équipée de godrons axiaux 27 qui sont en appui sur la paroi interne de la jupe extérieure 25 afin d'assurer un écartement maîtrisé entre les parois délimitant le deuxième conduit amont 23. En outre, des secteurs 28 de circulation du produit dans le deuxième conduit amont 23 sont formés entre les godrons 27. Ainsi, le deuxième conduit amont 23 est discontinu angulairement afin de limiter son volume.

Par ailleurs, les godrons 27 portent des joncs 30 d'encliquetage de la base 9 dans une gorge 31 formée dans la paroi interne de la jupe extérieure 25 en formant entre eux des secteurs 32 d'alimentation en produit du deuxième conduit amont 23.

En relation avec les figures 1 à 4, 7 et 8, les godrons 27 s'étendent sur l'extrémité libre de la cupule 21 en étant en appui sur la portée 26 pour former des secteurs 29 de circulation du produit dans la deuxième jonction 24 en U.

Sur les figures 1 à 3, le premier conduit amont 18 et le conduit aval 17 sont annulaires et présentent chacun un diamètre qui est inférieur au diamètre du deuxième conduit amont 23.

En relation avec les figures 4 et 5, le premier conduit amont 18 peut être discontinu angulairement en prévoyant qu'au moins certains godrons 27 s'étendent sur la paroi interne de la cupule 21. En particulier, il est alors possible de faire varier la section relative des canaux amont 18, 23 afin d'optimiser la perte de charge dans le chemin de restitution 2, notamment en prévoyant comme représenté que seul un godron 27 sur deux s'étende sur la paroi interne de la cupule 21.

## Revendications

1. Module de distribution d'un produit destiné à être monté sur un conduit (4) d'amenée sous pression dudit produit, ledit module présentant un chemin de restitution (2) du produit depuis un passage d'alimentation (5) destiné à être en communication avec ledit conduit (4)
vers un passage de sortie (3) dudit produit en vue de sa distribution, ledit chemin de restitution (2)
présentant au moins un conduit aval (17) qui débouche dans le passage de sortie (3) et au moins un conduit amont (18) qui est en communication avec le passage d'alimentation (5), les conduits amont (18) et aval (17) présentant une jonction (19) en U qui est agencée pour mettre lesdits conduits amont (18) et aval (17)
en communication, au moins l'un desdits conduits amont (18) et aval (17)
étant délimité par au moins une paroi qui est apte à assurer une action microbiocide sur le produit contenu dans ledit conduit, le chemin de restitution (2) présentant un deuxième conduit amont (23) dans lequel débouche le passage d'alimentation (5), les deux conduits amont (18, 23) étant en communication par une deuxième
jonction (24) en U dont la direction est opposée à celle de la jonction (19) en U mettant en communication les conduits amont (18) et aval (17), ledit module comprenant un chapeau (8) qui est monté sur une base (9) en formant entre eux le chemin de restitution (2) présentant les passages d'alimentation (5) et de sortie (3), le chapeau (8) présentant un orifice (10) débouchant dans une paroi (11), la base (9) présentant un pointeau (12) dont l'extrémité libre est disposée dans ledit orifice (10)
pour former le passage de sortie (3), la paroi (11) étant équipée d'une jupe intérieure (20) qui s'étend sous l'orifice (10) en étant disposée autour du pointeau (12) pour former le conduit aval (17) à l'interface entre la paroi interne de ladite jupe intérieure (20) et la paroi périphérique dudit pointeau (12), le conduit amont (18) étant formé à l'interface entre la paroi externe de ladite jupe intérieure (20)
et la paroi interne d'une cupule (21) de la base (9), la jonction (19) en U étant formée entre l'extrémité libre de la jupe intérieure (20) et une portée (22) de raccordement entre ledit pointeau et ladite cupule (21), la paroi (11) étant équipée d'une jupe extérieure (25) s'étendant autour de la jupe intérieure (20), le deuxième conduit amont (23) étant formé à l'interface entre la paroi externe de la cupule (21) et la paroi interne de la jupe extérieure (25), la deuxième jonction (24) en U étant formée entre l'extrémité libre de la cupule (21) et une portée (26) de raccordement entre lesdites jupes (20, 25),
**caractérisé en ce que** la paroi externe de la cupule (21) est équipée de godrons axiaux (27) qui sont en appui sur la paroi interne de la jupe extérieure (25) en formant entre eux des secteurs (28) de circulation du produit dans le deuxième conduit amont (23), les godrons (27) portant des joncs (30) d'encliquetage de la base (9) dans le chapeau (8).

2. Module de distribution selon la revendication 1, **caractérisé en ce que** le conduit aval (17) présente une direction ascendante de circulation du produit depuis la jonction (19) en U vers le passage de sortie (3), le conduit amont (18) présentant une direction descendante de circulation dudit produit.

3. Module de distribution selon l'une des revendications 1 ou 2, **caractérisé en ce que** les conduits amont (18) et aval (17) sont disposés concentriquement par rapport à un axe (A) de sortie du produit.

4. Module de distribution selon l'une quelconque des revendications 1 à 3, **caractérisé en ce que** le conduit amont (18) est disposé autour d'au moins une partie du conduit aval (17).

5. Module de distribution selon l'une quelconque des revendications précédentes, **caractérisé en ce que** le passage de sortie (3) débouche dans une paroi (11) qui est apte à assurer une action microbiocide sur le produit disposé sur ladite paroi (11) entre deux distributions.

6. Module de distribution selon l'une quelconque des revendications précédentes, **caractérisé en ce que** la paroi apte à assurer une action microbiocide est réalisée à base d'au moins un matériau présentant des propriétés microbiocides par diffusion d'un agent antimicrobien, par contact avec un agent microbiostatique et/ou par irradiation avec un rayonnement de longueur d'onde adaptée.

7. Récipient comprenant un réservoir (1) dans lequel un produit est destiné à être conditionné, ledit récipient présentant un conduit (4) d'amenée sous pression dudit produit et, montée sur ledit conduit (4) un module de distribution selon l'une quelconque des revendications précédentes.

8. Récipient selon la revendication 7, **caractérisé en ce qu'**il est sous la forme d'un tube composé d'une jupe souple (6) surmontée par une tête (7), ladite tête présentant un goulot dans lequel le conduit (4) d'amenée est formé et une épaule (7a) de liaison dudit goulot à la jupe souple (6).

## Patentansprüche

1. Ausgabemodul für ein Produkt, das dazu bestimmt ist, an einer Leitung (4) zur Zuführung des Produkts unter Druck angebracht zu werden, wobei das Modul einen Kanal (2) zur Rückführung des Produkts aus einem Zuführungsdurchlass (5) aufweist, der dazu bestimmt ist, mit der Leitung (4)
zu einem Austrittsdurchlass (3) für das Produkt im Hinblick auf dessen Abgabe in Verbindung zu stehen, wobei der Rückführungskanal (2)
mindestens eine stromabwärtige Leitung (17), die in den Austrittsdurchlass (3) mündet, und mindestens eine stromaufwärtige Leitung (18) aufweist, die mit dem Zuführungsdurchlass (5) in Verbindung steht, wobei die stromaufwärtige (18) und die stromabwärtige (17) Leitung eine U-förmige Verbindung (19) aufweisen,
die so angeordnet ist, dass sie die stromaufwärtige (18) und die stromabwärtige (17) Leitung in Verbindung bringt, wobei mindestens eine der stromaufwärtigen (18) und stromabwärtigen (17) Leitung durch mindestens eine Wand begrenzt ist, die in der Lage ist, eine mikrobizide Wirkung auf das in der Leitung enthaltene Produkt sicherzustellen, wobei der Rückführungskanal (2) eine zweite stromaufwärtige Leitung (23) aufweist, in die der Zuführungsdurchlass (5) mündet, wobei die beiden stromaufwärtigen Leitungen (18, 23)
durch eine zweite U-förmige Verbindung (24) verbunden sind, deren Richtung derjenigen der U-förmigen Verbindung (19) entgegengesetzt ist, indem sie die stromaufwärtige (18) und stromabwärtige (17) Leitung miteinander verbindet, wobei das Modul eine Kappe (8) umfasst, die auf einem Basisteil (9) aufgesetzt ist, indem sie zwischen ihnen den Rückführungskanal (2) mit dem Zuführungs- (5) und Austrittsdurchlass (3) bildet, wobei die Kappe (8) eine Öffnung (10) aufweist, die in eine Wand (11) mündet, das Basisteil (9) eine Kanüle (12) aufweist, deren freies Ende in der Öffnung (10) angeordnet ist,
um den Austrittsdurchlass (3) zu bilden, wobei die Wand (11) mit einer Innenschürze (20) versehen ist, die sich unter der Öffnung (10) erstreckt und um die Kanüle (12) herum angeordnet ist, um die stromabwärtige Leitung (17) an der Schnittstelle zwischen der Innenwand der Innenschürze (20) und der peripheren Wand der Kanüle (12) zu bilden, wobei die stromaufwärtige Leitung (18) an der Schnittstelle zwischen der Außenwand der Innenschürze (20)
und der Innenwand eines Bechers (21) des Basisteils (9) ausgebildet ist, wobei die U-förmige Verbindung (19) zwischen dem freien Ende der Innenschürze (20) und einer Anschlussauflagefläche (22) zwischen der Kanüle und dem Becher (21) ausgebildet ist, wobei die Wand (11) mit einer Außenschürze (25) versehen ist, die sich um die Innenschürze (20) herum erstreckt, wobei die zweite stromaufwärtige Leitung (23) an der Schnittstelle zwischen der Außenwand des Bechers (21) und der Innenwand der Außenschürze (25) ausgebildet ist, wobei die zweite U-förmige Verbindung (24) zwischen dem freien Ende des Bechers (21) und einer Anschlussauflagefläche (26) zwischen den Schürzen (20, 25) ausgebildet ist, **dadurch gekennzeichnet, dass** die Außenwand des Bechers (21) mit axialen Riffeln (27) versehen ist, die auf der Innenwand der Außenschürze (25) aufliegen und zwischen sich Sektoren (28) zum Fließen des Produkts in der zweiten stromaufwärtigen Leitung (23) bilden, wobei die Riffel (27) Rastringe (30) zum Einrasten des Basisteils (9) in der Kappe (8) besitzen.

2. Ausgabemodul nach Anspruch 1, **dadurch gekennzeichnet, dass** die stromabwärtige Leitung (17) eine aufwärts gerichtete Fließrichtung des Produkts von der U-förmigen Verbindung (19) zum Austrittsdurchlass (3) aufweist, während die stromaufwärtige Leitung (18) eine abwärts gerichtete Fließrichtung des Produkts aufweist.

3. Ausgabemodul nach einem der Ansprüche 1 oder 2, **dadurch gekennzeichnet, dass** die stromaufwärtige (18) und stromabwärtige (17) Leitung konzentrisch zu einer Produktaustrittsachse (A) angeordnet sind.

4. Ausgabemodul nach einem der Ansprüche 1 bis 3, **dadurch gekennzeichnet, dass** die stromaufwärtige Leitung (18) um mindestens einen Teil der stromabwärtigen Leitung (17) herum angeordnet ist.

5. Ausgabemodul nach einem der vorstehenden Ansprüche, **dadurch gekennzeichnet, dass** der Austrittsdurchlass (3) in eine Wand (11) mündet, die in der Lage ist, eine mikrobizide Wirkung auf das auf der Wand (11) angeordnete Produkt zwischen zwei Ausgaben zu gewährleisten.

6. Ausgabemodul nach einem der vorstehenden Ansprüche, **dadurch gekennzeichnet, dass** die Wand, die in der Lage ist, eine mikrobizide Wirkung zu gewährleisten, aus mindestens einem Material besteht, das durch Diffusion eines antimikrobiellen Mittels, durch Kontakt mit einem mikrobiostatischen Mittel und/oder durch Bestrahlung mit Strahlung einer geeigneten Wellenlänge mikrobizide Eigenschaften aufweist.

7. Behälter mit einem Reservoir (1), in dem ein Produkt verpackt werden soll, wobei der Behälter eine Leitung (4) zur Zuführung des Produkts unter Druck und ein auf der Leitung (4) aufgesetztes Ausgabemodul nach einem der vorstehenden Ansprüche umfasst.

8. Behälter nach Anspruch 7, **dadurch gekennzeichnet, dass** er die Form einer Tube aufweist, die aus einer biegsamen Schürze (6) besteht, auf die ein Kopf (7) aufgesetzt ist, wobei der Kopf einen Hals, in dem die Zuführungsleitung (4) ausgebildet ist, und eine Schulter (7a) zur Verbindung des Halses mit der biegsamen Schürze (6) aufweist.

## Claims

1. A module for dispensing a product intended to be mounted on a duct (4) for supplying said product under pressure, said module having a return path (2) for returning the product from a feed passage (5) intended to be in communication with said duct (4) to a passage (3) for the outlet of said product for dispensing it, said return path (2) having at least one downstream duct (17) which leads into the outlet passage (3) and at least one upstream duct (18) which is in communication with the feed passage (5), the upstream (18) and downstream (17) ducts having a U-shaped junction (19) which is designed to bring said upstream (18) and downstream (17) ducts into communication, at least one of said upstream (18) and downstream (17) ducts being delimited by at least one wall which is able to ensure a microbiocidal action on the product contained in said duct, the return path (2) having a second upstream duct (23) into which the feed passage (5) leads to, the two upstream ducts (18, 23) being in communication via a second U-shaped junction (24), the direction of which is opposite to that of the U-shaped junction (19) bringing the upstream (18) and downstream (17) ducts into communication, said module comprising a cap (8) which is mounted on a base (9) forming between them the return path (2) having the feed (5) and outlet (3) passages, the cap (8) having an orifice (10) leading into a wall (11), the base (9) having a needle (12), the free end of which is disposed in said orifice (10) to form the outlet passage (3), the wall (11) being equipped with an inner skirt (20) which extends under the orifice (10) and is disposed around the needle (12) to form the downstream duct (17) at the interface between the inner wall of said inner skirt (20) and the peripheral wall of said needle (12), the upstream duct (18) being formed at the interface between the outer wall of said inner skirt (20) and the inner wall of a cup (21) of the base (9), the U-shaped junction (19) being formed between the free end of the inner skirt (20) and a connecting surface (22) between said needle and said cup (21), the wall (11) being equipped with an outer skirt (25) extending around the inner skirt (20), the second upstream duct (23) being formed at the interface between the outer wall of the cup (21) and the inner wall of the outer skirt (25), the second U-shaped junction (24) being formed between the free end of the cup (21) and a connecting surface (26) between said skirts (20, 25)
**characterised in that** the outer wall of the cup (21) is equipped with axial gadroons (27) which rest on the inner wall of the outer skirt (25), forming between them sectors (28) for the circulation of the product in the second upstream duct (23), the gadroons (27) carrying rings (30) for snap-fitting the base (9) in the cap (8).

2. The dispensing module according to claim 1, **characterised in that** the downstream duct (17) has an upward direction of flow of the product from the U-shaped junction (19) towards the outlet passage (3), the upstream duct (18) having a downward direction of flow of said product.

3. The dispensing module according to one of claims 1 or 2, **characterised in that** the upstream (18) and downstream (17) ducts are disposed concentrically with respect to an output axis (A) of the product.

4. The dispensing module according to any one of claims 1 to 3, **characterised in that** the upstream duct (18) is disposed around at least part of the downstream duct (17).

5. The dispensing module according to any one of the preceding claims, **characterised in that** the outlet passage (3) leads into a wall (11) which is able to ensure a microbiocidal action on the product disposed on said wall (11) between two dispensings.

6. The dispensing module according to any one of the preceding claims, **characterised in that** the wall capable of ensuring a microbiocidal action is made of at least one material having microbiocidal properties by diffusion of an antimicrobial agent, by contact with a microbiostatic agent and/or by irradiation with radiation of suitable wavelength.

7. A container comprising a reservoir (1) wherein a product is intended to be packaged, said container having a duct (4) for supplying said product under pressure and, mounted on said duct (4), a dispensing module according to any one of the preceding claims.

8. The container according to claim 7, **characterized in that** it is in the form of a tube composed of a flexible skirt (6) surmounted by a head (7), said head having a neck in which the supply duct (4) is formed and a shoulder (7a) for connecting said neck to the flexible skirt (6).
